# EUROPÄISCHE PATENTSCHRIFT

(11) **EP 0 758 896 B1**
(45) Veröffentlichungstag und Bekanntmachung des Hinweises auf die Patenterteilung: **19.08.1998**
(21) Anmeldenummer: 95912161.7
(22) Anmeldetag: 24.02.1995
(51) Int. Cl.: A61K 31/52, A61K 47/00

(54) **TOPISCHE ZUBEREITUNG ENTHALTEND ACICLOVIR**
TOPICAL PREPARATION CONTAINING ACICLOVIR
PREPARATION TOPIQUE CONTENANT DE L'ACICLOVIR

(30) Priorität: 11.05.1994 DE 4416674
(43) Veröffentlichungstag der Anmeldung: 26.02.1997
(73) Patentinhaber: Dr. Rentschler GmbH & Co. Medizin KG, 88471 Laupheim (DE)
(72) Erfinder: PETSZULAT, Monika, D-88471 Laupheim (DE); WALCH, Hatto, D-88471 Laupheim-Baustetten (DE)
(74) Vertreter: Schwabe - Sandmair - Marx
(86) Internationale Anmeldenummer: EP9500684
(87) Internationale Veröffentlichungsnummer: WO9531201

(56) Entgegenhaltungen:
- EP-A- 0 044 543
- EP-A- 0 336 000
- EP-A- 0 394 928
- EP-A- 0 410 348
- WO-A-88/04938
- WO-A-94/05258

## Beschreibung

Die Erfindung betrifft topische Zubereitungen, die als Wirkstoff Aciclovir enthalten.

Insbesondere betrifft die Erfindung amphiphile pharmazeutische Zubereitungen zur topischen Anwendung, die zur Behandlung von Virusinfektionen der Haut oder Schleimhaut eingesetzt werden und als Wirkstoff 9-[(2-Hydroxyethoxy)methyl]guanin, bekannt als Aciclovir in mikronisierter Form oder dessen pharmazeutisch verträgliche Salze oder Ester enthalten.

Aciclovir sowie pharmazeutisch verwendbare Derivate hiervon sind Guanin-Derivate mit antiviralen Eigenschaften, die zur Prophylaxe und Therapie schwerer systemischer Virus-Infektionen bei angeborener Immunschwäche oder sekundären Immundefekten, wie sie z. B. im Verlauf der Aids-Erkrankung oder immunsuppresiven und/oder zytostatischen Behandlungen auftreten, angewendet werden, wenn eine Ansprechbarkeit gewährleistet ist, wie das z.B. bei Infektionen mit Herpes simplex-Viren Typ I und II der Fall ist. Insbesondere wird Aciclovir zur Behandlung von Herpes simplex eingesetzt, GB-A-15 23 865.

Aciclovir ist eine in Wasser schwer lösliche und in den meisten organischen Lösungsmitteln unlösliche Substanz.

Wichtig für die Entwicklung topischer Formulierungen ist die Herstellung stabiler Zubereitungen, die den Wirkstoff optimal an die Haut freigeben und gut physiologisch verträglich sind. Die Cremegrundlage ist dabei von entscheidender Bedeutung für die Therapie, da sie eine Eigenwirkung besitzt.

Aciclovirhaltige topische Formulierungen werden in der EP-A-44 543 beschrieben. In diesem Patent wird eine O/W-Emulsion beschrieben, die eine disperse Ölphase und eine kontinuierliche Wasserphase umfaßt, wobei die Formulierung mindestens 30 % (w/w) eines mehrwertigen Alkohols enthalten muß.

Es hat sich gezeigt, daß derartige O/W-Emulsionen oft instabil sind und von vielen Anwendern nicht optimal vertragen werden.

Die Anmelderin hat nun überraschend gefunden daß eine aciclovirhaltige topische Zubereitung auf Basis eines amphiphilen Systems, wobei der Wirkstoff in mikronisierter Form vorliegt, die vorgenannten Nachteile nicht aufweist, also insbesondere stabiler und besser verträglich ist als die bekannten topischen Formulierungen.

Die erfindungsgemäßen Zubereitungen enthalten als Basis ein amphiphiles System. Bei amphiphilen Systemen handelt es sich um Übergangsformen von O/W- und W/O Systemen, wobei Öl- und Wasserphase kontinuierlich vorliegen. Eine eindeutige Phasenzuordnung ist nicht mehr möglich.

Die erfindungsgemäßen Zubereitungen enthalten eine Fett-, eine Wasser- und eine Emulgatorphase sowie gegebenenfalls konsistenzgebende Stoffe. Die Wasserphase enthält neben Wasser noch den Alkoholanteil. Die Emulgatorphase enthält mindestens einen O/W und mindestens einen W/O Emulgator, wobei alle bekannten Emulgatoren des jeweiligen Typs verwendet werden können. Vorzugsweise werden nicht-ionogene Emulgatoren verwendet. Insbesondere vorteilhaft sind Glycerolmonostearate, Fettsäureester oder Fettalkoholether mit kurzkettigem PEG (Polymerisationsgrad 2 bis 3), Partialfettsäureester des Sorbitans und Polyoxyethylensorbitans und höhere Fettalkohole (C12-C18).

Besonders bevorzugt sind Emulgatoren, die unter der Warenbezeichnung Tegin®, Tagat®, Lanette®, Brij® und Myrij® kommerziell erhältlich sind.

Von den vorgenannten Substanzen sind wiederum besonders vorteilhaft Tegin®M, Tagat®S2 und Lanette®O.

Der Alkoholanteil umfaßt einen oder mehrere mehrwertige Alkohole oder Mischungen davon. Beispiele für den mehrwertigen Alkohol sind Propylenglykol, Glycerin, niedrigmolekulare Polyethlenglykole und 1,3-Butandiol. Bevorzugt ist Propylenglykol.

Beispiele für die konsistenzgebenden Stoffe sind Wachse, mittelkettige Trigylceride wie Miglyole®, pflanzliche Öle und Paraffine. Bevorzugt ist Miglyol®812.

Als Fettphasenbestandteile eignen sich übliche für topische Zubereitungen bekannte lipophile Substanzen wie Vaseline, Paraffine, Fette, Öle und Wachse. Bevorzugt ist Vaseline.

Darüberhinaus können die erfindungsgemäßen Zubereitungen, sofern erwünscht, weitere für topische Formulierungen bekannte Hilfs- und Zusatzstoffe enthalten.

Die erfindungsgemäßen Zubereitungen liegen üblicherweise als Creme vor.

In den erfindungsgemäßen Zubereitungen werden die Bestandteile in folgenden Konzentrationen eingesetzt:

Aciclovir von 1 bis 10 %, vorzugsweise 3 bis 5 % und am bevorzugtesten 5 %.
Vaseline 15 bis 50 %, vorzugsweise 20 bis 30 % und am bevorzugtesten 25 %.
Wasser 20 bis 40 %, vorzugsweise 30 bis 35 % und am bevorzugtesten 35 %.
Der mehrwertige Alkohol 10 bis 30 %, vorzugsweise 10 bis 20 % und am bevorzugtesten 10 %.
Die Emulgatorphase 10 bis 30 %, vorzugsweise 15 bis 20 % und am bevorzugtesten 17 %. Konsistenzgebende Stoffe von 0 bis 20 %, vorzugsweise 5 bis 15 % und am bevorzugtesten 8 %. Die Prozentangaben sind Gewichtsprozent.

In einer bevorzugten Ausführungsform enthält die Zubereitung 10 % mehrwertigen Alkohol, wobei insbesondere Propylenglykol bevorzugt ist.

Die Emulgatorphase enthält in einer bevorzugten Ausführungsform 17 %, wobei insbesondere eine Zubereitung mit 6 % Lanette O, 7 % Tagat S2 und 4 % Tegin M vorteilhaft ist.

Weiterhin enthält eine derartige vorteilhafte Ausführungsform 8 % konsistenzgebende Stoffe, insbesondere Miglyol 812.

Der Wasseranteil (ohne Alkohol) in einer derart bevorzugten Zubereitung liegt bei 35 % und der Vaselinegehalt bei 25 %.

In den erfindungsgemäßen Zubereitungen wird der Wirkstoff in mikronisierter Form mit einer Teilchengröße von 5 bis 50 µm, bevorzugt 5 bis 30 µm und insbesondere 10 bis 20 µm verwendet.

Die erfindungsgemäßen Zubereitungen werden durch die Leitfähigkeit als amphiphiles System charakterisiert.
Bekannterweise lassen sich O/W-Systeme, W/O-Systeme und amphiphile Systeme anhand der unterschiedlichen Leitfähigkeit unterscheiden (vgl. Dermatika, Hsg. Niedner, Ziegenmeyer; WVG Stuttgart 1992, S. 566 ff). Die amphiphile Natur der erfindungsgemäßen Zubereitun-gen wurde weiterhin durch die Polarisationsmikroskopie bestätigt.

Es wurde gefunden, daß die erfindungsgemäßen Zubereitungen eine deutlich verbesserte Stabilität im Vergleich zu den bekannten O/W-Zubereitungen besitzen.

Bei Therapieversuchen wurden die erfindungsgemäßen Zubereitungen von den Patienten als deutlich besser verträglich als die bekannten O/W-Emulsionen beschrieben.

Es zeigte sich auch, daß die erfindungsgemäßen Zubereitungen ohne Zusatz eines Konservierungsmittels ausreichend mikrobiologisch stabil sind.

### Beispiele:

### Beispiel 1: Herstellung einer erfindungsgemäßen Creme

| Zusammensetzung pro 1 g Creme: | |
|---|---|
| Aciclovir (mikronisiert) | 50,00 mg |
| Lanette® O | 60,00 mg |
| Vaseline | 250,00 mg |
| Tagat® S2 | 70,00 mg |
| Tegin® M | 40,00 mg |
| Miglyol® 812 | 80,00 mg |
| Propylenglykol | 100,00 mg |
| Wasser, gereinigt | 350,00 mg |
| Gesamt: | 1.000,00 mg |

Zur Herstellung der Creme werden die Fettphase und Emulgatorphase sowie der konsistenzgebende Stoff bei ca. 70° C aufgeschmolzen. Die auf 70° C erwärmte wässrige Phase, die auch den mehrwertigen Alkohol enthält, wird dann langsam unter ständigem Rühren zu der auf 70°C temperierten Fettphase zugegeben und emulgiert. Der Ansatz wird ca. 15 min. homogenisiert und anschließend auf etwa 45° C abgekühlt. In die so hergestellte amphiphile Trägerbasis wird Aciclovir unter ständigem Rühren eingetragen und nochmals homogenisiert. Nach dem Abkühlen der Creme auf Raumtemperatur wird diese, z.B. in Tuben abgefüllt.

### Beispiel 2: Messung der Leitfähigkeit der erfindungsgemäßen Zubereitung:

Die Leitfähigkeit der erfindungsgemäßen Zubereitung wurde im Vergleich zu definierten O/W-Emulsionen bzw. W/O-Emulsionen sowie im Vergleich zur Formulierung des Beispiels 1 der EP-A-44 543 gemessen. Die Vergleichszubereitung des Typs W/O ist eine wasserhaltige Wollwachsalkoholsalbe und die Vergleichszubereitung des Typs O/W ist eine wasserhaltige hydrophile Salbe (DAB 10, 10. Ausgabe 1991, 2. Nachtrag 1993, Band 4, Monographien P-Z). Die Messung der Leitfähigkeit erfolgte mit einem Universal-Digitalmeter (DiGi 610 mit Leitfähigkeitseinschub LF 610 E, Wissenschaftliche Technische Werkstätten GmbH Weilheim), bei 25°C.

Die Ergebnisse sind in der folgenden Tabelle gezeigt.

| Zubereitung | Leitfähigkeit (µS x cm⁻¹) |
|---|---|
| O/W | 60,5 |
| W/O | 0,2 |
| Beispiel 1 der EP-A-44 543 | 31,4 |
| Erfindungsgemäße Zubereitung gemäß Beispiel 1 | 5,0 |

Weitere Untersuchungen mit anderen erfindungsgemäßen Zubereitungen ergaben Leitfähigkeitswerte von 4 bis 30 µS x cm⁻¹.

### Beispiel 3: Untersuchung der Stabilität der erfindungsgemäßen Zubereitung:

Die Stabilität der erfindungsgemäßen Zubereitungen wurde im Vergleich zur Zubereitung des Beispiels 1 der EP-A-44 543 im Zentrifugentest (5000 upm, 10 min.) untersucht (vgl. Dermatika: a.a.O.). In dieser Untersuchung zeigte sich, daß im Gegensatz zur Zubereitung des Beispiels 1 der EP-A-44 543, die eine deutliche Phasentrennung erkennen ließ, die erfindungsgemäße Zubereitung stabil war.

### Beispiel 4: Therapieversuche:

Zur Untersuchung der Wirksamkeit und Verträglichkeit der erfindungsgemäßen Zubereitungen wurden Therapieversuche an Patienten mit Herpes labialis durchgeführt.

### Patient: A.B.

- Alter:: 62 Jahre
- Diagnose:: linsengroßes Rezidiv an der Oberlippe;
- Symptome:: Jucken, Kribbeln, Stechen, Brennen, Schmerzen und Rötung;
- Therapiedauer:: 6 d, 5 Appl./d;
- Zustand bei Beendigung der Therapie:: vollständige Abheilung, kein Erythem vorhanden;
- Anmerkung:: keine Nebenwirkung, gute lokale Verträglichkeit.

### Patient: T.W.

- Alter:: 23 Jahre
- Diagnose:: großes Rezidiv an der Oberlippe, beginnende Rötung;
- Symptome:: Jucken, Kribbeln, Stechen, Brennen, Schmerzen und Rötung;
- Therapiedauer:: 5 d, 4-5 Appl./d;
- Zustand bei Beendigung der Therapie:: vollständige Abheilung;
- Anmerkung:: bessere Verträglichkeit als die bekannten O/W-Zubereitungen bei gleicher Wirksamkeit, insbesondere weniger aggressiv, keinerlei Hautreizungen, keinerlei Hautrötungen sowie insbesondere gute Wirksamkeit bei Brennen und Schmerzen.

### Patient: J.H.

- Alter:: 31 Jahre
- Diagnose:: 1 cm großes Rezidiv an der Oberlippe;
- Symptome:: Jucken, Kribbeln, Stechen, Brennen, Schmerzen, Rötung, Bläschen;
- Therapiedauer:: 10 d, 4-5 Appl./d;
- Zustand bei Beendigung der Therapie:: vollständige Abheilung;

Zusammenfassend läßt sich zu den Therapieversuchen feststellen, daß auch bei weiteren Anwendungen festgestellt wurde, daß die erfindungsgemäßen Zubereitungen eine mindestens gleich gute Wirksamkeit wie die bekannten aciclovirhaltigen Cremes aufweisen, darüberhinaus jedoch von den Patienten als besser verträglich als die handelsüblichen O/W-Cremes beschrieben wurden und zu einer schnelleren Beseitigung der Schmerzen führen.

## Patentansprüche

1. Aciclovirhaltige topische Zubereitung, dadurch gekennzeichnet, daß der Wirkstoff in mikronisierter Form vorliegt und die Formulierung als amphiphiles System aufgebaut ist.

2. Zubereitung nach Anspruch 1, in der das amphiphile Trägersystem Vaseline, Wasser, mindestens einen O/W- und mindestens einen W/O-Emulgator und einen mehrwertigen Alkohol enthält.

3. Zubereitung nach Anspruch 2, in der der O/W- und der W/O-Emulgator aus der Gruppe der nicht-ionogenen Emulgatoren ausgewählt ist.

4. Zubereitung nach Anspruch 3, in der der O/W- und der W/O-Emulgator aus der Gruppe Glycerolmonostearate, Fettsäurester oder Fettalkoholether mit kurzkettigen PEG, Partialfettsäureester des Sorbitans und Polyoxyethylensorbitans und C₁₂-C₁₈-Fettalkohole ausgewählt ist.

5. Zubereitung nach Anspruch 4, in der die Emulgatoren aus der Gruppe Tegin, Tagat, Lanette, Brij und Myrij ausgewählt sind.

6. Zubereitung nach Anspruch 5, in der die Emulgatoren Tegin M®, Tagat®S2 und Lanette® O sind.

7. Zubereitung nach einem der Ansprüche 1 bis 6, in der der mehrwertige Alkohol aus Propylenglykol, Glycerin, 1,3-Butandiol, niedrigmolekularen PEG's und Mischungen davon ausgewählt ist.

8. Zubereitung nach Anspruch 7, in der der mehrwertige Alkohol Propylenglykol ist.

9. Zubereitung nach einem der Ansprüche 1 bis 8, in der der Wirkstoff in mikronisierter Form mit einer Teilchengröße von 5 bis 50 µm, vorzugsweise 5 bis 30 µm und insbesondere 10 bis 20 µm vorliegt.

10. Zubereitung nach einem der Ansprüche 1 bis 9, die 1 bis 10 %, vorzugsweise 3 bis 5 % Wirkstoff; 15 bis 50 %, vorzugsweise 20 bis 30 % Vaseline; 0 bis 20 %, vorzugsweise 5 bis 15 % konsistenzgebenden Stoff; 10 bis 30 %, vorzugsweise 10 bis 20 % mehrwertigen Alkohol; 20 bis 40 %, vorzugsweise 30 bis 35 % Wasser und 10 bis 30 %, vorzugsweise 15 bis 20 % Emulgator enthält, wobei die Angaben Gewichtsprozent sind.

11. Zubereitung nach Anspruch 10, die 5 % Wirkstoff, 25 % Vaseline, 17 % Emulgator, 10 % mehrwertigen Alkohol, 35 % Wasser und 8 % konsistenzgebende Stoffe enthält, wobei die Angaben Gewichtsprozent bedeuten.

12. Zubereitung nach Anspruch 11, die als Emulgator 6 % Lanette O, 7 % Tagat S2 und 4 % Tegin M enthält.

13. Verfahren zur Herstellung einer aciclovirhaltigen topischen Zubereitung nach einem der Ansprüche 1 bis 12, bei dem man die Fettphase und die Emulgatorphase und den konsistenzgebenden Stoff bei ca. 70°C aufschmilzt, die auf ca. 70°C erwärmte Wasserphase mit dem mehrwertigen Alkohol langsam unter ständigem Rühren zu der auf 70°C temperierten Fettphase zugibt, die Mischung emulgiert, anschließend für 10 bis 20 min, vorzugsweise 15 min, homogenisiert, die Mischung dann auf ca. 45°C abkühlt, das mikronisierte Aciclovir unter ständigem Rühren zugibt, nochmals homogenisiert und nach Abkühlen in die gewünschten Behältnisse abfüllt.

## Claims

1. Topical preparation containing Acyclovir, characterized in that the active ingredient is present in micronised form and the formulation is constructed as an amphiphilic system.

2. Preparation according to Claim 1, in which the amphiphilic vehicle system contains Vaseline, water, at least one oil/water emulsifer and at least one water/oil emulsifier, and a polyvalent alcohol.

3. Preparation according to Claim 2, in which the oil/water emulsifier and the water/oil emulsifier is chosen from the group of non-ionic emulsifiers.

4. Preparation according to Claim 3, in which the oil/water emulsifier and the water/oil emulsifier is chosen from the group glycerol monostearates, fatty acid esters or fatty alcohol ethers with short-chain PEG, partial fatty acid esters of sorbitan and polyoxyethylensorbitan and C₁₂-C₁₈ fatty alcohols.

5. Preparation according to Claim 4, in which the emulsifiers are chosen from the group Tegin, Tagat, Lanette, Brij and Myrij.

6. Preparation according to Claim 5, in which the emulsifiers are Tegin M®, Tagat®S2 and Lanette® O.

7. Preparation according to one of the Claims 1 to 6, in which the polyvalent alcohol is chosen from propylene glycol, glycerine, 1,3-butandiol, low molecular weight PEGs and mixtures thereof.

8. Preparation according to Claim 7, in which the polyvalent alcohol is propylene glycol.

9. Preparation according to one of the Claims 1 to 8, in which the active ingredient is present in micronised form with a particle size of 5 to 50 µm, preferably 5 to 30 µm and especially 10 to 20 µm.

10. Preparation according to one of the Claims 1 to 9, containing 1 to 10 %, preferably 3 to 5 % active ingredient; 15 to 50 %, preferably 20 to 30 % Vaseline; 0 to 20 %, preferably 5 to 15 % consistency-imparting substance; 10 to 30 %, preferably 10 to 20 % polyvalent alcohol; 20 to 40 %, preferably 30 to 35 % water and 10 to 30 %, preferably 15 to 20 % emulsifier, the amounts stated being weight percent.

11. Preparation according to Claim 10, containing 5 % active ingredient, 25 % Vaseline, 17 % emulsifier, 10 % polyvalent alcohol, 35 % water and 8 % consistency-imparting substances, the amounts stated being weight percent.

12. Preparation according to claim 11, containing as emulsifier 6 % Lanette O, 7 % Tagat S2 and 4 % Tegin M.

13. Process for the manufacture of a topical preparation containing Acyclovir according to one of the Claims 1 to 12 in which the fatty phase and the emulsifier phase and the consistency-imparting substance are melted together at approx. 70°C, the water phase with the polyvalent alcohol heated up to approx. 70°C are added slowly with continuous stirring to the fatty phase thermostated at 70°C, the mixture is emulsified, afterwards homogenised for 10 to 20 minutes, preferably 15 minutes, the mixture is then cooled down to approx. 45°C, the micronised Acyclovir added with continuous stirring, homogenised once more and, after cooling down, packed into the required containers.

## Revendications

1. Préparation topique contenant de l'acyclovir, caractérisée par le fait que le principe actif est présent sous forme micronisée et que la formulation est conçue sous la forme d'un système amphiphile.

2. Préparation selon la revendication 1, dans laquelle le système de support amphiphile contient de la vaseline, de l'eau, au moins un émulsifiant huile-dans-l'eau et au moins un émulsifiant eau-dans-l'huile, et un alcool à valences multiples.

3. Préparation selon la revendication 2, dans laquelle l'émulsifiant huile-dans-l'eau et l'émulsifiant eau-dans-l'huile sont choisis dans le groupe des émulsifiants non-ioniques.

4. Préparation selon la revendication 3, dans laquelle l'émulsifiant huile-dans-l'eau et l'émulsifiant eau-dans-l'huile sont choisis dans le groupe constitué par le monostéarate de glycérol, des esters d'acides gras ou des éthers d'alcools gras avec du PEG à courte chaîne, des esters partiels d'acides gras de sorbitan et des polyoxyéthylènes-sorbitan et des alcools gras en C₁₂-C₁₈.

5. Préparation selon la revendication 4, dans laquelle les émulsifiants sont choisis dans le groupe constitué par le Tegin, le Tagat, le Lanette, le Brij et le Myrij.

6. Préparation selon la revendication 5, dans laquelle les émulsifiants sont le Tegin M®, le Tagat®S2 et le Lanette®O.

7. Préparation selon l'une quelconque des revendications 1 à 6, dans laquelle l'alcool à valences multiples est choisi parmi le propylèneglycol, la glycérine, le 1,3-butanediol, les PEG de faible poids moléculaire et les mélanges de ceux-ci.

8. Préparation selon la revendication 7, dans laquelle l'alcool à valences multiples est le propylèneglycol.

9. Préparation selon l'une quelconque des revendications 1 à 8, dans laquelle le principe actif sous forme micronisée présente une taille de particules de 5 à 50 µm, de préférence de 5 à 30 µm et en particulier, de 10 à 20 µm.

10. Préparation selon l'une quelconque des revendications 1 à 9, qui contient 1 à 10 %, de préférence 3 à 5 % de principe actif ; 15 à 50%, de préférence 20 à 30% de vaseline ; 0 à 20%, de préférence 5 à 15 % d'épaississant ; 10 à 30 %, de préférence 10 à 20 % d'alcool à valences multiples ; 20 à 40 %, de préférence 30 à 35 % d'eau et 10 à 30 %, de préférence 15 à 20 % d'émulsifiant, les pourcentages étant exprimés en poids.

11. Préparation selon la revendication 10, qui contient 5 % de principe actif, 25 % de vaseline, 17 % d'émulsifiant, 10 % d'alcool à valences multiples, 35 % d'eau et 8 % d'épaississant, les pourcentages étant exprimés en poids.

12. Préparation selon la revendication 11, qui contient, en tant qu'émulsifiant, 6 % de Lanette, 0,7 % de Tagat S2 et 4 % de Tegin M.

13. Procédé d'obtention d'une préparation topique contenant de l'acyclovir, selon l'une quelconque des revendications 1 à 12, dans lequel on fait fondre la phase grasse et la phase d'émulsifiant et l'épaississant à environ 70°C, on introduit lentement, sous agitation constante, la phase aqueuse chauffée à environ 70°C avec l'alcool à valences multiples dans la phase grasse maintenue à 70°C, on émulsifie le mélange, ensuite on l'homogénéise pendant 10 à 20 minutes, de préférence pendant 15 minutes, ensuite on refroidit le mélange à environ 45°C, on introduit l'acyclovir micronisé sous agitation constante, on homogénéise à nouveau le mélange et après refroidissement, on le conditionne dans des conteneurs appropriés.
